# EUROPÄISCHE PATENTSCHRIFT

(11) **EP 4 305 640 B1**
(45) Veröffentlichungstag und Bekanntmachung des Hinweises auf die Patenterteilung: **30.10.2024**
(21) Anmeldenummer: 23711452.5
(22) Anmeldetag: 14.03.2023
(51) Int. Cl.: G16H 30/40, G16H 40/20, G16H 40/40

(54) **INSTRUMENTENTRACKING UND -INSPEKTION ZUR AUTOMATISCHEN IDENTIFIKATION MEDIZINISCHER/CHIRURGISCHER GEGENSTÄNDE**
INSTRUMENT TRACKING AND INSTRUMENT INSPECTION FOR AUTOMATICALLY IDENTIFYING MEDICAL/SURGICAL ITEMS
SUIVI D'INSTRUMENT ET INSPECTION D'INSTRUMENT POUR IDENTIFIER AUTOMATIQUEMENT DES ARTICLES MÉDICAUX/CHIRURGICAUX

(30) Priorität: 17.03.2022 DE 102022106281
(43) Veröffentlichungstag der Anmeldung: 17.01.2024
(73) Patentinhaber: Aesculap AG, 78532 Tuttlingen (DE)
(72) Erfinder: JANSEN-TROY, Arne, 78333 Stockach (DE); GOERZ, Dennis, 78532 Tuttlingen (DE); MAAS, Allan, 78467 Konstanz (DE); HENKE, Matthias, 78048 Villingen-Schwenningen (DE); KIESSLING, Daniel, 78502 Villingen-Schwenningen (DE)
(74) Vertreter: Winter, Brandl - Partnerschaft mbB
(86) Internationale Anmeldenummer: PCT/EP2023/056517
(87) Internationale Veröffentlichungsnummer: WO 2023/174955

(56) Entgegenhaltungen:
- EP-B1- 2 882 366
- CN-U- 214 865 378
- US-A1- 2007 007 339
- US-A1- 2020 082 934
- LELACHAICHAROEANPAN JAROONWIT ET AL: "Classification of Surgical Devices with Artificial Neural Network Approach", 2021 7TH INTERNATIONAL CONFERENCE ON ENGINEERING, APPLIED SCIENCES AND TECHNOLOGY (ICEAST), IEEE, 1 April 2021 (2021-04-01), pages 154 - 159, XP033914190, DOI: 10.1109/ICEAST52143.2021.9426258
- DIANA MARTINS LAVADO: "Sorting Surgical Tools from a Cluttered Tray -Object Detection and Occlusion Reasoning Imagem", 28 September 2018 (2018-09-28), XP055670808, Retrieved from the Internet <URL:https://estudogeral.uc.pt/bitstream/10316/86257/1/Tese-Diana_VersaoFinal.pdf> [retrieved on 20200221]

## Beschreibung

Die vorliegende Offenbarung betrifft eine Vorrichtung zum automatischen Identifizieren von medizinischen und/ oder chirurgischen Gegenständen mit einer Erfassungseinrichtung, welche zumindest eine Scannereinheit und einen Detektor aufweist.

### Technisches Gebiet

Im Gesundheitswesen geht der Trend immer deutlicher in Richtung Einzelinstrumententracking, um auf Instrumenten- OP-Materialebene die jeweiligen medizinischen und/ oder chirurgischen Gegenstände verfolgen zu können, insbesondere um die Vollständigkeit der Gegenstände post- aber auch intraoperativ überprüfen zu können. Solche Vollständigkeitskontrollen dienen ferner dazu, einen großen und kostenintensiven Schwund an medizinischen und/ oder chirurgischen Gegenständen zu reduzieren sowie die Patientensicherheit zu erhöhen.

Der Aspekt der Patientensicherheit gewinnt vor dem Hintergrund an Bedeutung, dass bei einer Notfalloperation manchmal etwa 100 Druckrollen und 40 Kompressen neben den sonstigen Instrumenten zum Einsatz kommen. Ferner ist aus Krankenhausstatistiken bekannt, dass jährlich durchschnittlich 3000 Fälle auftreten, in denen Fremdkörper wie Tücher, Tupfer oder ähnliches nach chirurgischen Eingriffen im Körper des Patienten verbleiben.

Neben traditionellen manuellen Methoden wie Einsortieren und Zählen kommen mittlerweile verschiedenste Technologien zu Anwendung, welche die Erfassung von chirurgischen Instrumenten unterstützen oder sogar vollautomatisiert ablaufen lassen.

### Stand der Technik

Der Stand der Technik zum Tracking von chirurgischen Instrumenten beinhaltet beispielsweise die RFID-Technologie, weitere Funksysteme wie beispielsweise Bluetooth und optische maschinelle Lesesysteme wie Barcodes, Datamatrixcodes etc. und klassische vom Menschen lesbare alphanummerische Daten sowie Systeme, die kombinierte Technologien nutzen.

Beispiele zu der RFID-Technologie offenbaren die PCT/EP2017/062129, PCT/GB2015/052977, PCT/IB2020/055731, PCT/US2012/030046. Die Verwendung von Bluetooth ist beispielsweise in PCT/CN2014/095452 beschrieben.

Derzeit etablieren sich Künstliche Intelligenz (KI)--basierte Tracking-Technologien, wie beispielsweise Video- und Laser-Scannersysteme, zum Erkennen sowie u.a. Klassifizieren und Zuordnen von Objekten. Vorreiter in diesem Segment sind Entwickler selbstfahrender Fahrzeuge und robotischer Industriesysteme. Ebenfalls sind KI-basierte radiologische Systeme bekannt für Ultraschall und fluoroskopische Bildgebungsverfahren zur Optimierung der Diagnose. Intraoperativ kommen ebenfalls Trackingsysteme zum Einsatz, die zum Ziel haben die Position von zuvor definierten Instrumenten bzw. deren Endeffektoren zu erfassen.

Das Auslesen mithilfe von RFID-Technik zeigt, dass in der Regel nicht 100% aller Instrumente erfasst werden können und außerdem technische, aber auch regulatorische Anforderungen das Aufbringen von RFID-Tags auf Bestandsinstrumente erschwert.

Das Auslesen mithilfe von Barcodes oder Datamatrixcodes kann die Eingabe bzw. die Zuordnung von Artikelnummern erheblich erleichtern, kann derzeit aber auch nicht vollständig automatisiert erfolgen. Kontrastarme, korrodierte oder zerkratzte Codes erschweren zudem das Ein- bzw. Auslesen oder machen dies teilweise unmöglich.

Optische Verfahren zur Erkennung von Objekten werden bereits auch zum Identifizieren bzw. Tracking von chirurgischen Instrumenten eingesetzt, dabei werden diese aus verschiedenen Blickwinkeln vor eine Kamera, in einigen Fällen Stereokameras oder 3D-Kamerasysteme, gehalten und mithilfe einer KI werden diese Objekte eingelernt, um später automatisch erkannt zu werden. Im Fall von chirurgischen Instrumenten werden diese vereinzelt auf eine optisch homogene Oberfläche gelegt und die Lichtverhältnisse konstant gehalten. So können die Objekte erkannt werden - auch verschiedene, abweichende Geometrien und Größen können differenziert werden und entsprechend einer hinterlegten Artikelnummer zugeordnet werden.

Diese optische Technik stößt an ihre Grenzen, sobald sich die Lichtverhältnisse stark ändern oder Reflexionen auftreten, in diesen Fällen können die Objekte nicht mehr eindeutig zugeordnet werden. Insbesondere wenn viele Instrumente chaotisch übereinandergelegt werden, kann das System die Instrumente nicht mehr differenzieren. Versuche haben ebenfalls gezeigt, dass Instrumente, welche beispielsweise in eine Schale gelegt werden, durch die veränderten optischen Bedingungen, insbesondere Reflexionen und schwache Kontraste nicht mehr eindeutig erkannt bzw. zugeordnet werden können.

Bei einer anderen Inspektionsgeräteart steht die Qualitätsprüfung von gefertigten elektronischen Bauteilen im Vordergrund, um beispielsweise Lötstellen und Durchkontaktierung im Inneren von Platinen zu prüfen. Hier können Bildauflösungen von 500 nm mit 3D-CT Scannern, in der Regel nach dem Laminographie-Verfahren, erreicht werden. Es können so topographische Eigenschaften dargestellt werden. Im Gegensatz zu den Röntgeninspektionsgeräten der Lebensmittelindustrie verbleiben die Prüflinge bei diesem Verfahren lange Zeit und ohne Änderung der Position im Scanner.
EP 2 882 366 B1 offenbart eine Einrichtung mit einer ersten Erfassungseinrichtung mit einer ersten Erfassungstechnologie zum Erfassen von Instrumenten und Instrumenteninformationen, insbesondere einer RFID-Erkennung, und einer zweiten Erfassungseinrichtung mit einer zweiten, von der ersten verschiedenen Erfassungstechnologie zum Erfassen von Instrumenten und Instrumenteninformationen sowie einer Abgleicheinrichtung zum Abgleichen der Erfassungsergebnisse der beiden Erfassungstechnologien.
CN 214 865 378 U offenbart eine automatische Erkennungs- und Sortierausrüstung mit einer Abschirmbox, einer Fördervorrichtung zum Fördern von zu sortierenden Objekte; einer Erfassungseinrichtung zur Erfassung von Bildinformationen der zu sortierenden Objekte und einer Sortiervorrichtung zum Sortieren der Objekte.
Weiterer Stand der Technik ist aus der US 2020/082934 A1 sowie der Nichtpatentliteratur "Classification of Surgical Devices with Artificial Neural Network Approach", 2021 7TH INTERNATIONAL CONFERENCE ON ENGINEERING, APPLIED SCIENCES AND TECHNOLOGY (ICEAST), IEEE, 1. April 2021, Seiten 154-159, XP033914190.
US 2007/007339 A1 offenbart ein Verfahren und eine Vorrichtung zum Sortieren und Lagern von chirurgischen Instrumenten, wobei die Vorrichtung Röntgenstrahlen verwendet. Kurzbeschreibung der Offenbarung

Vor dem Hintergrund ist die objektiv technische Aufgabe der vorliegenden Offenbarung, eine Vorrichtung bereitzustellen, welche die vorstehenden Nachteile beseitigt oder zumindest verbessert.

Diese Aufgabe wird gelöst durch die Aspekte des Anspruchs 1.

Demnach betrifft die vorliegende Offenbarung eine Vorrichtung zum automatischen, gleichzeitigen, Identifizieren von einer Vielzahl und weiter bevorzugt von unterschiedlichen, medizinischen und/ oder chirurgischen Gegenständen, mit einer Erfassungseinrichtung, welche zumindest eine Scannereinheit und einen Detektor aufweist bzw. verwendet und/oder so verwendet, dass sich medizinische und/ oder chirurgische Gegenstände zumindest kurzzeitig während der automatischen und gleichzeitigen Identifizierung zwischen der Scannereinheit und dem Detektor befinden, und einer vorzugsweise an/in einem Gehäuse angeordneten Bildgebungs- und Recheneinheit, wobei die zumindest eine Scannereinheit eine Röntgenquelle ist oder hat und die Rechen- und Bildgebungseinheit dazu vorgesehen und ausgebildet ist, um die Vollständigkeit der medizinischen und/ oder chirurgischen Gegenstände und/ oder das Fehlen zumindest eines medizinischen und/oder chirurgischen Gegenstands, vorzugsweise post- und/oder intraoperativ, zu erkennen und auszugeben und/oder medizinische und/ oder chirurgische Gegenstände nicht nur auf Artikelnummernebene sondern auch auf Chargenebene zu erkennen.

In anderen Worten basiert die vorliegende Offenbarung auf einer analogen Hardware von bekannten Röntgeninspektionsgeräten, insbesondere den Systemen, welche über eine Scannereinheit, vorzugsweise einen Zeilenscanner mit einem Förderband für die zu inspizierende Ware verfügen. In der Regel ist die Scannereinheit als Röntgenquelle mit Bildgebungseinheit sowie einer Rechnereinheit mit entsprechender Software zur Bildgebung ausgebildet.

In nochmals anderen Worten können mithilfe der Röntgenstrahlung zur Bildgebung in Kombination mit einer Software insbesondere chaotisch angeordnete medizinische und/ oder chirurgische Gegenstände einzeln identifiziert und somit getrackt/nachverfolgt werden. Das heißt wiederum, dass insbesondere die Vollständigkeit der Gegenstände unmittelbar post- aber auch intraoperativ geprüft werden kann und gegebenenfalls das Fehlen von chirurgischen Instrumenten automatisiert über ein Mensch-Maschine-Interface angezeigt werden kann. Hier kann neben einer erhöhten Patientensicherheit auch ein verbesserter Informationsgehalt bezüglich verlorener Gegenstände/ Instrumente zu einem sehr frühen Zeitpunkt beim Rücklauf in eine Aufbereitungseinheit für Medizinprodukte (AEMP) geschaffen werden. Derzeit ist nur eine manuelle intraoperative Zählkontrolle der Instrumente und Materialien mithilfe von abzuarbeitenden Listen bekannt. Ein automatisiertes Verfahren gemäß der vorliegenden Offenbarung, vereinfacht hier die Qualitätssicherung deutlich und ebenfalls kann ein zeitlicher Aufwand von intraoperativ verlorenen Gegenständen beschleunigt werden, da auch klar darstellbar ist, welche Gegenstände gegebenenfalls nicht vorhanden sind. Des Weiteren können menschliche Fehler vermieden werden.

Wiederum in anderen Worten ist die Röntgeninspektionstechnologie ausschlaggebend. Vergleichbare Geräte kommen vor allem bei der Inspektion von Gepäck und Postpaketen zum Einsatz, um explosive oder andere illegale Stoffe aufzuspüren.

Bei der Inspektionstechnologie für Lebensmittel sollen vor allem Fremdkörper wie Messerabbrüche oder sonstige Metalle sowie Knochenstücke, Glas oder Kunststoff teilweise in der fertigen Verpackung detektiert werden. Auch in anderen Produkten aus Kunststoff, Holz oder Textilien wird mit der Technologie nach Fremdkörpern gesucht, die im eigentlichen Produkt nicht vorkommen sollen und die Produkte bzgl. weiterer Merkmale überprüft. Im Vordergrund der etablierten Technik steht aber die Detektion von Fremdkörper, nicht aber deren Größe oder Lage. Es ist Stand der Technik, dass die Röntgeninspektionsgeräte auch mit weiteren Systemen wie Waagen und optischen Systemen kombiniert werden, um weitere gewünschte Produkteigenschaften wie Gewicht und Form zu verifizieren. Die Röntgeninspektionsgeräte verfügen in der Regel über ein Förderband, welches die zu kontrollierenden Gegenstände automatisch durch die Scanner befördert. Bei den Geräten können, im Fall von Lebensmittelverpackungen, Durchlaufgeschwindigkeiten von 1,5 m/s erreicht werden bei einer Bildauflösung von 0,4 mm und 150 bis 255 Graustufen.

Weitere Aspekte werden gemäß den Unteransprüchen nachfolgend näher beschrieben.

Es ist bevorzugt, wenn ein Gehäuse die vorstehenden Komponenten, insbesondere die Erfassungseinrichtung sowie die zu identifizierenden medizinischen und/ oder chirurgischen Gegenstände umgibt.

Es ist bevorzugt, wenn zwei Erfassungseinheiten vorgesehen sind, welche vorzugsweise orthogonal zueinander angeordnet sind.

Es ist von Vorteil, wenn ein Förderband zwischen der zumindest einen ersten Scannereinheit und dem Detektor derart vorgesehen und angeordnet ist, um die medizinischen und/oder chirurgischen Gegenstände auf dem Förderband zu transportieren. Die medizinischen und/oder chirurgischen Gegenstände können vereinzelt sein, sich aber auch in einem Transportbehältnis wie einem Siebkorb und/oder Sterilcontainer befinden. Ferner ragt das Förderband vorzugsweise beim Ein- und Auslass über das Gehäuse hinaus.

Des Weiteren ist es von Vorteil, wenn eine erste Scannereinheit in fixer Position orthogonal zu dem Förderband angeordnet ist.

In anderen Worten sind verschiedene Scanner-Aufbauten vorgesehen, beispielsweise als Zeilenscanner mit Förderband (mit entsprechender Peripherie wie zumindest einem röntgenundurchlässigen Vorhang), als Flächenscanner mit und ohne Förderband sowie Variationen der Scannereinheiten-Anzahl, wobei hierbei zwei Scannereinheiten in orthogonaler Anordnung oder in der Position variierbar, vorteilhafter Weise aber nur eine Scannereinheit in fixer Position in orthogonaler Scanrichtung zum Förderband vorgesehen sind. Unter einem Zeilenscanner ist hierbei ein Scanner zu verstehen, dessen Kernelement eine CCD-Zeile ist, die von einem Schrittmotor in Mikroschritten über einen Gegenstand transportiert wird und somit Zeile für Zeile abgetastet wird. Im Gegensatz hierzu tastet der Flächenscanner eine Fläche ab.

Es ist bevorzugt, wenn ein röntgenundurchlässiger Vorhang vorgesehen ist, welcher derart angeordnet ist, um die zumindest eine Scannereinheit als auch die medizinischen und/ oder chirurgischen Gegenstände während der Identifizierung von der Umgebung abzuschirmen. Die Röntgenstrahlung wird dabei durch Vorhänge über dem Förderband aus entsprechendem Material von der Umgebung abgeschirmt.

Es ist von Vorteil, wenn die Darstellung der medizinischen und/ oder chirurgischen Gegenstände durch die Rechen- und Bildgebungseinheit in Echtzeit erfolgt. In anderen Worten wird mithilfe eines Monitors die durchlaufende Ware/ die medizinischen und/ oder chirurgischen Gegenstände in Echtzeit dargestellt.

Bevorzugter Weise ist eine Sortiereinheit vorgesehen, welche dazu vorgesehen und ausgebildet ist, um die medizinischen und/ oder chirurgischen Gegenstände automatisiert ihrer aktuellen Lage entsprechend zu entnehmen und/ oder zu vereinzeln und einem Folgeprozess zuzuführen. In anderen Worten ist es vorgesehen, dass die vorliegende Offenbarung ebenfalls in der AEMP einsetzbar ist. Hier kann mithilfe der Bilddaten ein räumliches Modell aller chaotisch angeordneten Instrumente im Verbund erstellt werden, welches für eine robotische Einheit nutzbar ist, um die Gegenstände/ Instrumente automatisiert ihrer aktuellen Lage entsprechend zu entnehmen bzw. zu vereinzeln und den Folgeprozessen zuzuführen.

Zudem kann die vorliegende Vorrichtung des Weiteren über einen integrierten automatischen Auswurfmechanismus von nicht-konformer Ware/ konformen medizinischen und/ oder chirurgischen Gegenständen verfügen.

Es ist bevorzugt, wenn die Bildgebungs- und Recheneinheit dazu vorgesehen und ausgebildet ist, um über eine Schnittstelle zum Abgleichen der identifizierten medizinischen und/ oder chirurgischen Gegenstände auf eine entsprechende Gegenstandsliste zuzugreifen und die identifizierten medizinischen und/ oder chirurgischen Gegenstände mit dieser abzugleichen. In anderen Worten kann eine Schnittstelle auf entsprechende Instrumenten- und/oder Materiallisten zurückgreifen und diese abgleichen.

Es ist von Vorteil, wenn eine künstliche-Intelligenz-Einrichtung basierend auf zuvor erstellter 3D-Daten von den medizinischen und/ oder chirurgischen Gegenständen eine Identifikation erleichtert. In anderen Worten soll mithilfe von zuvor erstellten 3D-Daten von medizinischen und/ oder chirurgischen Gegenständen durch eine KI eine Identifikation insbesondere nur durch eine Ansicht durch ein röntgenbasiertes Bildgebungsverfahren (d.h. in der Regel durch die Draufsicht auf die längsten Achsen der Instrumente) genügen. Die Identifikation erfolgt über den Abgleich zuvor erlernter röntgenbasierter Aufnahmen, die im Vergleich zu optischen Systemen mit einer sehr hohen Reproduzierbarkeit aufgrund der Eliminierung von äußeren Störgrößen, wie Änderungen der Lichtintensität, Reflexionen, Farbgebung etc., durchgeführt werden kann. Es wird vorgeschlagen, dass die Identifikation der medizinischen und/ oder chirurgischen Gegenstände dabei nicht nur auf Artikelnummernebene stattfinden kann, sondern auch auf Chargenebene, wenn entsprechende Unterschiede in der Geometrie, beispielweise hervorgerufen durch Form- und Lagetoleranzen der einzelnen medizinischen und/ oder chirurgischen Gegenstände, eine eindeutige Zuordnung ermöglichen.

Ferner betrifft die vorliegende Offenbarung ein Identifizierungssystem, mit einer Vielzahl medizinischer und/ oder chirurgischer Gegenstände, einer Erfassungseinrichtung zum automatischen und gleichzeitigen Identifizieren von der Vielzahl medizinischer und/ oder chirurgischer Gegenständen, welche zumindest eine Scannereinheit und einen Detektor aufweist bzw. verwendet, und eine Bildgebungs- und Rechnereinheit, welche vorzugsweise an/in einem Gehäuse angeordnet ist, wobei die zumindest eine Scannereinheit eine Quelle der Röntgenstrahlung ist oder hat und die Rechen- und Bildgebungseinheit dazu vorgesehen und ausgebildet ist, um die Vollständigkeit der medizinischen und/ oder chirurgischen Gegenstände und/ oder das Fehlen zumindest eines medizinischen und/oder chirurgischen Gegenstands, vorzugsweise post- und/oder intraoperativ, zu erkennen und auszugeben.

Insbesondere ist es bevorzugt, wenn das System mit weiteren Systemen wie Warenwirtschaftsprogramm-Systemen kommuniziert. Hier können mithilfe der Software schon im Vorfeld weitere Stellen in der AEMP benachrichtigt werden, falls Instrumente unauffindbar bleiben und ersetzt werden müssen. Derzeit wird ein Verlust von Instrumenten erst beim Abarbeiten der Packlisten beim Bestücken der Siebkörbe in der AEMP ermittelt, d.h. mit dem vorgeschlagenen System können just-in-time Warenbereitstellungen durchgeführt werden.

Des Weiteren ist eine Kontrolle bezüglich Beschädigungen von Instrumenten bspw. Mängel von Schneiden-Geometrien oder innere mechanische Komponenten, wie sie bei Drehmomentbegrenzungssystemen vorkommen, möglich. Diese Mängel können bevorzugt automatisiert angezeigt werden.

Insbesondere ist es bevorzugt, wenn sich alle zu identifizierenden Gegenstände in einem für Röntgenstrahlung, aber nicht unbedingt für sichtbares Licht, durchlässigen verschlossenen Behälter befinden. Der Behälter soll insbesondere automatisiert das Röntgeninspektionsgerät durchlaufen und die zuvor genannten Informationen mit Peripheriegeräten mittels der Schnittstelle austauschen. Hier wird das Verletzungsrisiko für die Anwender minimiert, da die Instrumente für die Zählkontrolle nicht erneut gehandhabt werden müssen.

Des Weiteren soll mithilfe des 3D-Modells der Gegenstände/ Instrumente eine KI ermitteln, ob Instrumente ineinandergesteckt sind und entsprechend eine Strategie zur problemlosen Entnahme ermittelt werden. Mithilfe der Röntgenbildgebungsverfahren ist hier eine Darstellung möglich, welche mit optischem Licht bei steigender Anzahl an Instrumenten nicht mehr möglich ist.

In allen vorstehenden Fällen ist eine Umhausung/ ein Gehäuse bzw. Gehäuse mit Vorhang/ Vorhängen der Röntgenquelle, der Sensoren und der zu inspizierenden Behälter und Gegenstände/ Instrumente vorgesehen.

Im Idealfall sind die Parameter der Röntgenbildgebungskomponenten für die Nutzung mit einem Sterilcontainer und entsprechenden Sieben ausgelegt. Hierbei sind entsprechende softwareseitige Filter zur Bildgebung vorgesehen.

### Kurzbeschreibung der Figuren

Fig. 1 ist eine Darstellung zur Veranschaulichung einer Vorrichtung bzw. eines Systemaufbaus gemäß einer ersten Ausführungsform der vorliegenden Offenbarung;
Fig. 2 ist eine Darstellung zur Veranschaulichung einer Vorrichtung bzw. eines Systemaufbaus gemäß einer zweiten Ausführungsform der vorliegenden Offenbarung; und
Fig. 3 ist eine Darstellung zur Veranschaulichung der Kommunikation zwischen den Komponenten.

### Beschreibung der Ausführungsformen

Nachstehend werden Ausführungsbeispiele der vorliegenden Offenbarung auf der Basis der zugehörigen Figuren beschrieben.

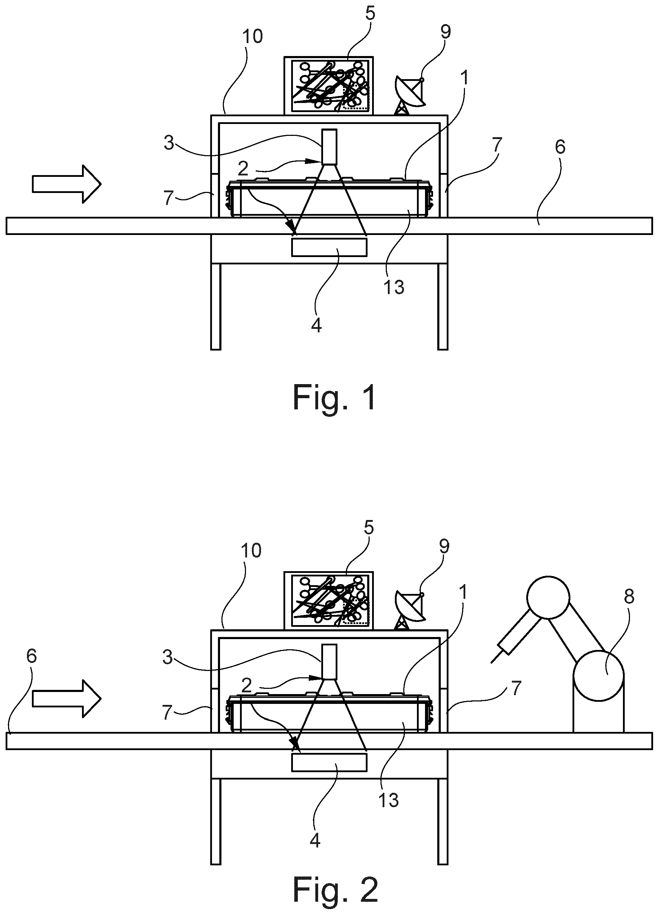

Fig. 1 ist eine Darstellung zur Veranschaulichung einer Vorrichtung bzw. eines Systemaufbaus gemäß einer ersten Ausführungsform der vorliegenden Offenbarung. Die Vorrichtung bzw. das System zum automatischen Identifizieren von medizinischen und/ oder chirurgischen Gegenständen 1 hat eine Erfassungseinrichtung 2. Die Erfassungsvorrichtung 2 weist zumindest eine Scannereinheit 3 und einen Detektor 4 auf. Um eine (automatische) Identifikation durchführen zu können, müssen sich die medizinischen und/ oder chirurgischen Gegenstände 1 zumindest kurzzeitig - während der automatischen Identifizierung - zwischen der Scannereinheit 3 und dem Detektor 4 befinden. Ferner weist die Vorrichtung eine vorzugsweise an/in einem Gehäuse 10 angeordneten Bildgebungs- und Recheneinheit 5 auf. Die Bildgebungs- und Recheneinheit 5 hat einen Monitor, auf welchem die Darstellung eines entsprechenden Bildes darstellbar ist.

Die zumindest eine Scannereinheit 3 ist eine Quelle elektromagnetischer Strahlung, erfindungsgemäß Röntgenstrahlung. Die Rechen- und Bildgebungseinheit 5 ist dazu vorgesehen und ausgebildet, um die Vollständigkeit der medizinischen und/ oder chirurgischen Gegenstände 1 und/ oder das Fehlen zumindest eines medizinischen und/oder chirurgischen Gegenstands 1, vorzugsweise post- und/oder intraoperativ, zu erkennen und auszugeben.

Ferner ist in Fig. 1 ein Förderband 6 zwischen der Scannereinheit 3 und dem Detektor 4 derart vorgesehen und angeordnet, um die medizinischen und/oder chirurgischen Gegenstände 1 auf dem Förderband 6 zu transportieren. Hierbei ist es bevorzugt, wenn die Scannereinheit 3 in fixer Position orthogonal zu dem Förderband 2 angeordnet ist. In anderen Worten ist der Detektor 4 unterhalb des Förderbands 6, vorzugsweise mittig von dem Gehäuse 10, vorgesehen. Die Scannereinheit 3 ist innerhalb des Gehäuses 10 oberhalb des Förderbands 6 angeordnet. Die Röntgenquelle der Scannereinheit 3 ist in Richtung hin zu dem Detektor 4 ausgerichtet und ist vorzugsweise mittig zu dem Detektor 4 angeordnet.

Durch die Bewegung des Förderbands 6 in Richtung des dargestellten Pfeils der Fig. 1 werden die medizinischen und/oder chirurgischen Gegenstände 1 zwischen der Scannereinheit 3 und dem Detektor 4 hindurch transportiert. Auf diese Weise können alle auf dem Förderband 6 befindlichen Gegenstände, insbesondere OP-Materialien und chirurgische Instrumente, in Echtzeit auf dem Monitor der Bildgebungs- und Recheneinheit 5 dargestellt werden.

Die medizinischen und/oder chirurgischen Gegenstände 1 sind vorzugsweise in einem Siebkorb und/oder Sterilcontainer 13 oder einem anderen für elektromagnetische Strahlung, erfindungsgemäß Röntgenstrahlung durchlässigen Behälter vorgesehen.

Ein röntgenundurchlässiger Vorhang 7 ist derart angeordnet, um die zumindest eine Scannereinheit 3 als auch die medizinischen und/ oder chirurgischen Gegenstände 1 während der Identifizierung von der Umgebung abzuschirmen. Der Vorhang 7 ist an dem Gehäuse 20 auf der Seite des Ein- und Auslasses des Förderbandes 6 vorgesehen. Die Seiten des Gehäuses 10, welche sich, zumindest abschnittsweise, parallel zu dem Förderband 6 erstrecken sind, dienen gleichzeitig als Abschirmung.

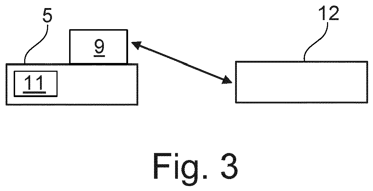

Ferner ist an dem Gehäuse 10 eine Schnittstelle 9 vorgesehen. Die Bildgebungs- und Recheneinheit 5 ist dazu vorgesehen und ausgebildet, um über eine Schnittstelle 9 zum Abgleichen der identifizierten medizinischen und/ oder chirurgischen Gegenstände 1 auf eine entsprechende Gegenstandsliste, vorzugsweise auf einem Peripheriegerät 12 (siehe Fig. 3) abgelegt, zuzugreifen und die identifizierten medizinischen und/ oder chirurgischen Gegenstände 1 mit dieser abzugleichen.

Fig. 2 ist eine Darstellung zur Veranschaulichung einer Vorrichtung bzw. eines Systemaufbaus gemäß einer zweiten Ausführungsform der vorliegenden Offenbarung. Die zweite Ausführungsform entspricht der ersten Ausführungsform mit dem Unterschied, dass die zweite Ausführungsform für eine Aufbereitungseinheit für Medizinprodukte (AEMP) vorgesehen ist. Aus dem Grund ist eine Sortiereinheit 8 vorgesehen.

Die Sortiereinheit 8 ist an einem Ende (in Pfeilrichtung) des Förderbands 6 vorgesehen. Ferner ist die Sortiereinheit 8 dazu vorgesehen und ausgebildet, um die medizinischen und/ oder chirurgischen Gegenstände 1 automatisiert ihrer aktuellen Lage entsprechend zu entnehmen und/ oder zu vereinzeln und einem Folgeprozess zuzuführen. Hierbei ist es bevorzugt, dass die Sortiereinheit 8 mit der Bildgebungs- und Recheneinheit 5 kommunizieren kann, um ein entsprechendes Eingreifen durch die Bildgebungs- und Recheneinheit 5 zu initiieren.

Fig. 3 ist eine Darstellung zur Veranschaulichung der Kommunikation zwischen den Komponenten. Die Vorrichtung bzw. das System weist eine künstliche-Intelligenz-Einrichtung 11 auf, die dazu vorgesehen und ausgebildet ist, um basierend auf zuvor erstellter 3D-Daten von den medizinischen und/ oder chirurgischen Gegenständen 1 eine Identifikation und einen Abgleich zu erleichtern. Die künstliche-Intelligenz-Einrichtung 11 ist vorzugsweise ein Teil der Bildgebungs- und Recheneinheit 5.

Die vorgesehene Kommunikation mit einem Peripheriegerät 12 erfolgt über die Schnittstelle 9, vorzugsweise über Bluetooth, Funk oder anderer gängiger Übertragungstechnologien.

### Bezugszeichen

- 1: medizinische und/ oder chirurgische Gegenstände
- 2: Erfassungseinrichtung
- 3: Scannereinheit
- 4: Detektor
- 5: Bildgebungs- und Recheneinheit
- 6: Förderband
- 7: Vorhang
- 8: Sortiereinheit
- 9: Schnittstelle
- 10: Gehäuse
- 11: Künstliche-Intelligenz-Einrichtung
- 12: Peripheriegerät
- 13: Siebkorb/ Sterilcontainer

## Patentansprüche

1. Vorrichtung zum automatischen und gleichzeitigen Identifizieren von mehreren medizinischen und/ oder chirurgischen Gegenständen (1) mit einer Erfassungseinrichtung (2), welche zumindest eine Scannereinheit (3) und einen Detektor (4) aufweist und/oder so verwendet, dass sich mehrere medizinische und/ oder chirurgische Gegenstände (1) zumindest kurzzeitig während der automatischen und gleichzeitigen Identifizierung zwischen der Scannereinheit (3) und dem Detektor (4) befinden, und mit einer vorzugsweise an/in einem Gehäuse (10) angeordneten Bildgebungs- und Recheneinheit (5), wobei die zumindest eine Scannereinheit (3) eine Quelle einer Röntgenstrahlung ist oder hat und die Rechen- und Bildgebungseinheit (5) dazu vorgesehen und ausgebildet ist, um die Vollständigkeit der mehreren medizinischen und/ oder chirurgischen Gegenstände (1) und/ oder das Fehlen zumindest eines medizinischen und/oder chirurgischen Gegenstands (1), vorzugsweise post- und/oder intraoperativ, zu erkennen und auszugeben.

2. Vorrichtung gemäß Anspruch 1, wobei zwei Erfassungseinheiten (2) vorgesehen sind, welche vorzugsweise orthogonal zueinander angeordnet sind.

3. Vorrichtung gemäß Anspruch 1 oder 2, wobei ein Förderband (6) zwischen der zumindest einen ersten Scannereinheit (3) und dem Detektor (4) derart vorgesehen und angeordnet ist, um die mehreren medizinischen und/oder chirurgischen Gegenstände (1) auf dem Förderband (6) zu transportieren.

4. Vorrichtung gemäß Anspruch 3, wobei die zumindest eine erste Scannereinheit (3) in fixer Position orthogonal zu dem Förderband (2) angeordnet ist.

5. Vorrichtung gemäß einem der Ansprüche 1 bis 4, wobei ein röntgenundurchlässiger Vorhang (7) vorgesehen ist, welcher derart angeordnet ist, um die zumindest eine Scannereinheit (3) als auch die mehreren medizinischen und/ oder chirurgischen Gegenstände (1) während der Identifizierung von der Umgebung abzuschirmen.

6. Vorrichtung gemäß einem der Ansprüche 1 bis 5, wobei die Darstellung der mehreren medizinischen und/ oder chirurgischen Gegenstände (1) durch die Rechen- und Bildgebungseinheit (5) in Echtzeit erfolgt.

7. Vorrichtung gemäß einem der Ansprüche 1 bis 6, wobei eine Sortiereinheit (8) vorgesehen ist, welche dazu vorgesehen und ausgebildet ist, um die mehreren medizinischen und/ oder chirurgischen Gegenstände (1) automatisiert ihrer aktuellen Lage entsprechend zu entnehmen und/ oder zu vereinzeln und einem Folgeprozess zuzuführen.

8. Vorrichtung gemäß einem der Ansprüche 1 bis 7, wobei die Bildgebungs- und Recheneinheit (5) dazu vorgesehen und ausgebildet ist, um über eine Schnittstelle (9) zum Abgleichen der identifizierten mehreren medizinischen und/ oder chirurgischen Gegenstände (1) auf eine entsprechende Gegenstandsliste, vorzugsweise auf einem Peripheriegerät (12) abgelegt, zuzugreifen und die identifizierten mehreren medizinischen und/ oder chirurgischen Gegenstände (1) mit dieser abzugleichen.

9. Vorrichtung gemäß einem der Ansprüche 1 bis 8, wobei eine künstliche-Intelligenz-Einrichtung (11) dazu vorgesehen und ausgebildet ist, um basierend auf zuvor erstellter 3D-Daten von den mehreren medizinischen und/ oder chirurgischen Gegenständen (1) eine Identifikation und einen Abgleich zu erleichtern.

10. Identifizierungssystem, mit:
einer Vielzahl medizinischer und/ oder chirurgischer Gegenstände (1),
einer Erfassungseinrichtung (2) zum automatischen und gleichzeitigen Identifizieren von der Vielzahl medizinischer und/ oder chirurgischer Gegenständen (1), welche zumindest eine Scannereinheit (3) und einen Detektor (4) aufweist, und
eine Bildgebungs- und Rechnereinheit (5), welche vorzugsweise an/in einem Gehäuse (10) angeordnet ist, wobei
die zumindest eine Scannereinheit (3) eine Röntgenquelle ist und die Rechen- und Bildgebungseinheit (5) dazu vorgesehen und ausgebildet ist, um die Vollständigkeit der Vielzahl medizinischen und/ oder chirurgischen Gegenstände (1) und/ oder das Fehlen zumindest eines medizinischen und/oder chirurgischen Gegenstands (1), vorzugsweise post- und/oder intraoperativ, zu erkennen und auszugeben.

## Claims

1. Device for automatic and simultaneous identification of several medical and/or surgical objects (1) comprising a detection device (2) which includes and/or uses at least one scanner unit (3) and one detector (4) in such a way that several medical and/or surgical objects (1) are located between the scanner unit (3) and the detector (4) at least for a short time during automatic and simultaneous identification, and an imaging and computing unit (5) preferably arranged on/in a housing (10), wherein the at least one scanner unit (3) is or has a source of X-ray radiation, and the computing and imaging unit (5) is provided and configured to detect and output, preferably post- and/or intraoperatively, the completeness of the plurality of medical and/or surgical objects (1) and/or the absence of at least one medical and/or surgical object (1).

2. The device according to claim 1, wherein two detection units (2) are provided, which are preferably arranged orthogonally to each other.

3. The device according to claim 1 or 2, wherein a conveyor belt (6) is provided and arranged between the at least one first scanner unit (3) and the detector (4) in such a way as to transport the plurality of medical and/or surgical objects (1) on the conveyor belt (6).

4. The device according to claim 3, wherein the at least one first scanner unit (3) is arranged in a fixed position orthogonal to the conveyor belt (2).

5. The device according to any of claims 1 to 4, wherein an X-ray-impermeable curtain (7) is provided, which is arranged so as to shield the at least one scanner unit (3) as well as the plurality of medical and/or surgical objects (1) from the environment during identification.

6. The device according to any one of claims 1 to 5, wherein the representation of the plurality of medical and/or surgical objects (1) by the computing and imaging unit (5) is carried out in real time.

7. The device according to any of claims 1 to 6, wherein a sorting unit (8) is provided, which is intended and configured to automatically remove and/or separate the plurality of medical and/or surgical objects (1) according to their current position and feed them to a subsequent process.

8. The device according to any of claims 1 to 7, wherein the imaging and computing unit (5) is provided and configured to access a corresponding object list, preferably stored on a peripheral device (12), via an interface (9) for matching the identified plurality of medical and/or surgical objects (1) and to match the identified plurality of medical and/or surgical objects (1) with this list.

9. The device according to any of claims 1 to 8, wherein an artificial intelligence device (11) is intended and configured to facilitate identification and matching based on previously created 3D data of the plurality of medical and/or surgical objects (1).

10. An identification system, comprising:
a plurality of medical and/or surgical objects (1),
a detection device (2) for automatically and simultaneously identifying the plurality of medical and/or surgical objects (1), including at least one scanner unit (3) and one detector (4), and
an imaging and computing unit (5), which is preferably arranged on/in a housing (10), wherein
the at least one scanner unit (3) is an X-ray source, and the computing and imaging unit (5) is provided and configured to recognize and output, preferably post- and/or intraoperatively, the completeness of the plurality of medical and/or surgical objects (1) and/or the absence of at least one medical and/or surgical object (1).

## Revendications

1. Dispositif pour l'identification automatique et simultanée de plusieurs objets médicaux et/ou chirurgicaux (1) avec un appareil de détection (2) qui présente au moins une unité de scanner (3) et un détecteur (4) et/ou qui les utilise de sorte que plusieurs objets médicaux et/ou chirurgicaux (1) se trouvent au moins brièvement entre l'unité de scanner (3) et le détecteur (4) pendant l'identification automatique et simultanée, et avec une unité d'imagerie et de calcul (5) disposée de préférence au niveau de/dans un boîtier (10), dans lequel la au moins une unité de scanner (3) est ou présente une source de rayons X et l'unité de calcul et d'imagerie (5) est prévue et conçue pour reconnaître et délivrer en sortie, de préférence en postopératoire et/ou peropératoire l'intégralité des plusieurs objets médicaux et/ou chirurgicaux (1) et/ou l'absence d'au moins un objet médical et/ou chirurgical (1).

2. Dispositif selon la revendication 1, dans lequel sont prévues deux unités de détection (2) qui sont de préférence disposées orthogonalement l'une par rapport à l'autre.

3. Dispositif selon la revendication 1 ou 2, dans lequel une bande de transport (6) est prévue et disposée entre la au moins une première unité de scanner (3) et le détecteur (4) de manière à transporter les plusieurs objets médicaux et/ou chirurgicaux (1) sur la bande de transport (6).

4. Dispositif selon la revendication 3, dans lequel la au moins une première unité de scanner (3) est disposée dans une position fixe orthogonale à la bande de transport (2).

5. Dispositif selon l'une quelconque des revendications 1 à 4, dans lequel est prévu un rideau radio-opaque (7) qui est disposé de manière à isoler de l'environnement la au moins une unité de scanner (3) ainsi que les plusieurs objets médicaux et/ou chirurgicaux (1) pendant l'identification.

6. Dispositif selon l'une quelconque des revendications 1 à 5, dans lequel la représentation des plusieurs objets médicaux et/ou chirurgicaux (1) par l'unité de calcul et d'imagerie (5) est effectuée en temps réel.

7. Dispositif selon l'une quelconque des revendications 1 à 6, dans lequel est prévue une unité de tri (8) qui est prévue et conçue pour enlever et/ou individualiser de manière automatisée les plusieurs objets médicaux et/ou chirurgicaux (1) en fonction de leur position actuelle et les amener à un processus consécutif.

8. Dispositif selon l'une quelconque des revendications 1 à 7, dans lequel l'unité d'imagerie et de calcul (5) est prévue et conçue pour accéder à une liste d'objets correspondante, de préférence stockée sur un périphérique (12), par l'intermédiaire d'une interface (9) pour comparer les plusieurs objets médicaux et/ou chirurgicaux (1) identifiés et pour comparer les plusieurs objets médicaux et/ou chirurgicaux (1) identifiés avec cette liste.

9. Dispositif selon l'une quelconque des revendications 1 à 8, dans lequel un appareil d'intelligence artificielle (11) est prévu et conçu pour faciliter une identification et une comparaison des plusieurs objets médicaux et/ou chirurgicaux (1) sur la base de données 3D préalablement créée.

10. Système d'identification avec :
une pluralité d'objets médicaux et/ou chirurgicaux (1),
un appareil de détection (2) pour l'identification automatique et simultanée de la pluralité d'objets médicaux et/ou chirurgicaux (1), appareil présente au moins une unité de scanner (3) et un détecteur (4), et
une unité d'imagerie et de calcul (5) qui est de préférence disposée au niveau de/dans un boîtier (10), dans lequel
la au moins une unité de scanner (3) est une source de rayons X et l'unité de calcul et d'imagerie (5) est prévue et conçue pour détecter et délivrer en sortie, de préférence en postopératoire et/ou peropératoire, l'intégralité de la pluralité d'objets médicaux et/ou chirurgicaux (1) et/ou l'absence d'au moins un objet médical et/ou chirurgical (1).
